Europäisches Patentamt

European Patent Office

Office européen des brevets

(19)

‖‖‖‖‖‖‖‖‖‖‖‖‖‖‖‖‖‖‖‖‖‖‖‖‖‖‖‖‖‖‖‖‖

(11) Numéro de publication : **0 387 140 B1**

## (12) FASCICULE DE BREVET EUROPEEN

(45) Date de publication du fascicule du brevet :
**08.06.94 Bulletin 94/23**

(51) Int. Cl.$^5$ : **A61K 31/615,** A61K 31/60,
// (A61K31/60, 31:185)

(21) Numéro de dépôt : **90400605.3**

(22) Date de dépôt : **06.03.90**

---

(54) **Composition à activité anti-agrégante plaquettaire utilisable en thérapeutique.**

---

(30) Priorité : **10.03.89 FR 8903138**

(43) Date de publication de la demande :
**12.09.90 Bulletin 90/37**

(45) Mention de la délivrance du brevet :
**08.06.94 Bulletin 94/23**

(84) Etats contractants désignés :
**AT BE CH DE DK ES FR GB GR IT LI LU NL SE**

(56) Documents cités :
**CHEMICAL ABSTRACTS, vol. 83, no. 13, 29 septembre 1975, Columbus, OH (US); K. HORI-SAKA et al., pp. 35-36, no. 108332n
CHEMICAL ABSTRACTS, vol. 73, no. 9, 31 août 1970, Columbus, OH (US); K. MORISAKA et al., p. 154, no. 43479n**

(56) Documents cités :
**ROTE LISTE, 1961, Editio Cantor-Aulendorf, Württemberg (DE); p. 396
CHEMICAL ABSTRACTS, vol. 95, no. 7, 17 août 1981, Columbus, OH (US); T. KIMURA et al., p. 41, no. 54819d
CHEMICAL ABSTRACTS, vol. 94, no. 13, 30 mars 1981, Columbus, OH (US); T. KIMURA et al., p. 158, no. 97860v**

(73) Titulaire : **PROCTER & GAMBLE PHARMACEUTICALS FRANCE
70, rue du Gouverneur Général Eboué
F-92130 Issy les Moulineaux (FR)**

(72) Inventeur : **Covarrubias, Jésus
Via G. Bechi, 3
I-Florence (IT)**

(74) Mandataire : **L'Helgoualch, Jean et al
Cabinet Sueur et L'Helgoualch
78, rue Carnot
F-95240 Cormeilles-en-Parisis (FR)**

EP 0 387 140 B1

## Description

La présente invention concerne une nouvelle composition utilisable en thérapeutique humaine et vétérinaire, et plus particulièrement une nouvelle composition à base d'acide acétylsalicylique et de taurine, ou de leurs sels, utilisable notamment comme anti-agrégant plaquettaire.

On connait le rôle des plaquettes dans le processus athérogène, et ses conséquences telles que les thromboses artérielles ou veineuses sur plaque d'athérosclérose (Wu et al. The Lancet, 2 (1974) p.924). Divers médicaments destinés à inhiber les fonctions plaquettaires ont été utilisés, tels que l'acide acétylsalicylique, le dipyridamole ou la ticlopidine. Ils ne sont cependant pas satisfaisants car ils provoquent des effets secondaires indésirables, ou n'offrent qu'une efficacité limitée.

L'acide acétylsalicylique (aspirine) est bien connu en thérapeutique où il est utilisé comme principe actif de médicaments en raison de ses propriétés pharmacologiques, notamment antipyrétiques, anti-inflammatoires et analgésiques. On a également constaté récemment que l'aspirine possède une activité antithrombotique par blocage de la synthèse du thromboxane A2 à partir de l'acide arachidonique, utile pour la prévention de l'infarctus du myocarde, dans le cas d'une administration régulière de doses comprises généralement entre 100 mg et 1 g par jour environ. On a également constaté que l'administration régulière d'aspirine, même à faibles doses (100 mg/jour) interfère dans la synthèse du $PGI_2$.

Toutefois, la protection procurée par un tel traitement n'est pas complète, comme indiqué par J.D. Folts et al. The Lancet (23 avril 1988) p.937-38. En outre, des effets inverses peuvent être observés lorsque les sujets traités sont des fumeurs. L'aspirine est également contre-indiquée pour le traitement de patients souffrant d'hypertension, car elle peut interférer avec les médicaments antihypertenseurs. Enfin, l'aspirine n'exerce pas d'action favorable sur l'adhérence des plaquettes à l'épithélium de l'artère. L'effet anti-agrégant de l'aspirine peut être inefficace, réduit, voire complètement masqué dans le cas de patients présentant des facteurs de risque tels que l'hypertension, l'hypercholesterolémie, le diabète et le tabagisme. Enfin, l'utilisation de l'aspirine peut s'accompagner d'effets secondaires tels que des irritations gastro-intestinales, ou des hémorragies, en particulier pendant des périodes prolongées d'administration.

Parmi les divers sels connus d'acide acétylsalicylique, des sels dérivés de taurine tels que la N,N-diacétylsalicyloyloxyéthyl taurine et une 3-N-acyloxyéthyl taurine où le groupe acyle est dérivé de l'acide acétylsalicylique, sont connus, par exemple dans les brevets FR 2.482.095 et FR 2.465.717. Ces composés présentent une activité analgésique, antipyrétique et anti-inflammatoire, ainsi qu'une meilleure compatibilité gastro-intestinale que l'acide acétylsalicylique.

La taurine est un acide aminé de faible toxicité, dont les propriétés ont été décrites dans de nombreuses publications. On sait que les plaquettes sanguines contiennent de la taurine qui inhibe l'agrégation plaquettaire induite par l'ADP (adénosine diphosphate) et le PAF (plasminogen activating factor). En outre, la taurine favorise la formation de $PGI_2$. Ainsi, l'utilisation de la taurine pour le traitement de l'insuffisance cardiaque a-t-elle été décrite par divers auteurs.

K. Horisaka et al. (Chem. Abst. vol.83 (1975) n°108332n) décrivent l'utilisation simultanée d'aspirine et de taurine, et constatent une augmentation des propriétés analgésiques et antipyrétiques bien connues de l'aspirine. Les mêmes auteurs (Chem. Abst. vol.73 (1970) n°43479n) observent que la glycine possède un effet d'accélération de l'absorption de l'aspirine dans l'estomac. Une composition pharmaceutique à activité analgésique, antipyrétique et antirhumatismale contenant de l'aspirine et de la glycine, est décrite dans Rote Liste (2 mars 1961) p.396. Toutefois, la possibilité de préparer une composition à activité anti-agrégante plaquettaire par une combinaison spécifique d'aspirine et de taurine n'est pas envisagée.

De nombreuses recherches ont été entreprises pour tenter de mettre au point des substances ayant une activité du type anti-agrégant plaquettaire meilleure et une application plus large que les médicaments connus.

La présente invention, résultant des travaux et expérimentations effectués par le demandeur, a pour objet une nouvelle composition à base d'acide acétylsalicylique et de taurine, ou de leurs sels, en combinaison, procurant une action du type anti-agrégant plaquettaire améliorée par rapport aux médicaments connus.

L'invention a encore pour objet une composition comprenant essentiellement une combinaison d'acide acétylsalicylique et de taurine, ou de leurs sels respectifs, dans un rapport en poids acide acétylsalicylique / taurine compris entre 1/5 et 1/50, en une quantité pharmacologiquement efficace, associés le cas échéant à un ou plusieurs excipients pharmaceutiquement acceptables, utile pour le traitement préventif des maladies cardiaques résultant d'une agrégation plaquettaire excessive, telles que les thromboses artérielles ou artériolaires, notamment dans le cadre de l'insuffisance coronaire (angine de poitrine, infarctus du myocarde), l'artériopathie périphérique, et la pathologie ischémique cérébrale.

Les expérimentations effectuées avec la nouvelle composition selon la présente invention ont montré que l'activité du type anti-agrégant plaquettaire de l'acide acétylsalicylique est potentialisée dans la combinaison avec la taurine, tandis que les effets secondaires indésirables de l'acide acétylsalicylique sont fortement réduits.

Suivant une caractéristique avantageuse, on a en outre constaté que la combinaison de taurine et d'acide acétylsalicylique conforme à la présente invention procure une protection contre les facteurs de risque contre lesquels l'acide acétylsalicylique isolément n'a aucun effet, et permet alors d'agir efficacement dans le traitement de patients tels que des fumeurs, des sujets hypertendus, et des diabétiques, pour lesquels le traitement à l'acide acétylsalicylique peut être contre-indiqué ou ne procurer que de médiocres résultats.

Les études toxicologiques effectuées sur la combinaison conforme à la présente invention, par comparaison avec la taurine et l'acide acétylsalicylique séparément, ont montré que la toxicité est inférieure à celle de l'acide acétylsalicylique en raison de la moindre quantité d'acide présente dans la combinaison et de l'absence de toxicité de la taurine.

On a constaté que l'action recherchée est la plus efficace lorsque le rapport en poids de l'acide acétylsalicylique à la taurine est compris entre 1/5 et 1/50, et de préférence entre 1/10 et 1/30.

Ainsi, selon une caractéristique de la présente invention, la composition est constituée d'une combinaison d'acide acétylsalicylique et de taurine, ou de leurs sels respectifs, dans un rapport en poids compris entre 1/5 et 1/50, et de préférence entre 1/10 et 1/30. Bien que l'acide acétylsalicylique et la taurine puissent être combinés dans la même composition, conformément à la présente invention, ils peuvent aussi être administrés séparément. Dans les deux cas, il est préférable que l'acide acétylsalicylique soit présent à faibles doses, et par exemple des doses unitaires de 20 à 50 mg conviennent.

L'administration peut être faite suivant les techniques classiques, le principe actif constitué par la combinaison de la taurine et de l'acide acétylsalicylique étant mélangé, si nécessaire, avec des excipients usuels pharmaceutiquement acceptables. Il est particulièrement avantageux d'administrer la composition suivant l'invention par voie orale, sous forme de comprimé ou de gélule. Suivant les techniques galéniques usuelles, on peut par exemple préparer des gélules contenant des granulés d'acide acétylsalicylique enrobés et de la taurine dans les proportions de 30 mg d'acide pour 400 mg de taurine. Ces proportions peuvent bien entendu être modifiées, dans le domaine indiqué ci-dessus, mais on utilise généralement des doses unitaires contenant 20 à 50 mg d'acide acétylsalicylique pour 400 à 500 mg de taurine. Il est préférable de veiller à ce que l'acide acétylsalicylique ne réagisse pas avec la taurine pour former un sel, bien qu'une telle réaction n'ait pas d'effet néfaste important sur l'activité de la composition de l'invention.

Les excipients usuels peuvent être utilisés dans la présente invention, et on peut les choisir par exemple parmi le polyéthylène glycol, le stéarate de mgnésium, le talc et le lactose.

La posologie est déterminée par le médecin en fonction de l'état du patient, et peut être par exemple de 1 ou 2 gélules comme ci-dessus par jour.

Par exemple, un traitement de prévention peut consister à administrer une gélule contenant 30mg d'acide acétylsalicylique et 500 mg de taurine une ou deux fois par jour, pendant une période aussi longue que nécessaire, et généralement sans limitation de durée. La posologie est la même que ci-dessus pour les patients présentant un risque particulier d'infarctus du myocarde.

Les exemples suivants illustrent l'invention plus en détail et montrent l'effet de synergie procuré par la composition conforme à la présente invention.

Effet anti-agrégant plaquettaire.

Des plaquettes extraites d'échantillons de plasma sanguin provenant de quatre patients sont utilisées pour déterminer l'activité anti-agrégante de la taurine, de l'acide acétylsalicylique, et de la combinaison suivant l'invention, en comparaison.

L'agrégation est provoquée par l'acide arachidonique (1,4 M) et l'adénosine diphosphate (3,0 M).

Le taux d'agrégation plaquettaire est mesuré dans un échantillon ne contenant aucun anti-agrégant (standard), puis pour les deux composés séparés (taurine 40 M, acide acétylsalicylique 10 M), et enfin pour la combinaison suivant l'invention. Les quantités de substances qui induisent ou inhibent l'agrégation sont exprimées en micromoles. Les résultats des tests sont exprimés en pourcentage d'inhibition par rapport à la quantité d'agrégant.

Les résultats sont indiqués au tableau ci-après.

| Essai | Standard | Taurine | Asp | Tau + Asp (40/10) |
|-------|----------|---------|-----|-------------------|
| 1 | 77 | 0 | 50 | 73 |
| 2 | 83 | 0 | 10 | 65 |
| 3 | 89 | 0 | 55 | 100 |
| 4 | 92 | 0 | 5 | 85 |

Ces résultats démontrent l'effet de synergie procuré par la combinaison suivant la présente invention. S'agissant de résultats in vitro, ils ne permettent cependant pas de déduire précisément le rapport acide acétylsalicylique / taurine à utiliser dans la composition conforme à l'invention.

On constate que dans le cas de la taurine isolément, l'inhibition est pratiquement inexistante aux doses testées, ce qui signifie que la synthèse de thromboxane A2 induit par l'acide arachidonique n'est pas bloquée.

Dans le cas de l'acide acétylsalicylique isolément, la capacité d'inhibition est confirmée, mais le pourcentage obtenu est médiocre et irrégulier d'un cas à l'autre.

Au contraire, la combinaison conforme à la présente invention procure un pourcentage d'inhibition élevé et plus uniforme, qui met en évidence l'effet de synergie. Dans ce cas, on constate que des plaquettes ne réagissant pas à l'aspirine isolément, réagissent à la combinaison de l'invention.

Essai clinique.

L'essai est réalisé sur un groupe de 9 patients mâles, fumeurs et hypertendus légers, d'âge supérieur à 55 ans, sans condition pathologique.

Ils sont répartis en 3 groupes: aux patients du groupe A, on administre de l'acide acétylsalicylique à raison de 30 mg par jour. On administre aux patients du groupe B de la taurine à raison de 1 g par jour. On administre aux patients du groupe C un mélange conforme à l'invention de 30 mg d'acide acétylsalicylique et de 1 g de taurine. Un groupe de contrôle de 3 patients (groupe D) ayant les mêmes ages reçoit 1 g de placebo. Les trois groupes présentent les mêmes facteurs de risque.

Le traitement est prolongé pendant 3 semaines. En début et en fin de traitement, on effectue des tests in vitro permettant de mesurer l'agrégation plaquettaire: ADP, collagène et acide arachidonique, formation de thromboxane seul et en présence d'adrénaline. On détermine également le taux de fibrinogène plasmatique et d'adrénaline.

Les résultats montrent que dans le groupe A ne recevant que l'acide acétylsalicylique, la formation de thromboxane induite par l'acide arachidonique est inhibée. Toutefois, les pourcentages d'inhibition d'agrégation induite par l'ADP et le collagène sont moins élevés que dans les groupes C et D. Le taux de fibrinogène plasmatique, supérieur à celui du groupe D, est inchangé en fin de traitement par rapport au début.

Dans le groupe B, la formation de thromboxane n'est pas inhibée. Les taux d'inhibition d'agrégation induite par l'ADP et le collagène sont inférieurs à ceux des groupes A et C. Les niveaux de fibrinogène plasmatique et d'adrénaline diminuent entre le début et la fin du traitement.

De meilleurs résultats sont obtenus dans le groupe C pour chaque paramètre mesuré. Les taux de fibrinogène et d'adrénaline dans le plasma ne sont pas statistiquement différents de ceux du groupe B.

Il est important de noter qu'aucun effet secondaire inhérent à l'aspirine n'a été observé au cours du traitement.

Ces résultats montrent que la combinaison de taurine et d'acide acétylsalicylique conforme à la présente invention est un anti-agrégant et un antithrombotique dont l'effet est potentialisé par rapport aux substances isolément, et qui agit non seulement contre l'agrégation plaquettaire, mais aussi contre les facteurs externes inducteurs de thrombose.

## Revendications

**Revendications pour les Etats contractants suivants : AT, BE, CH, DE, DK, FR, GB, IT, LI, LU, NL, SE**

1. Composition utile en thérapeutique, exerçant une activité anti-agrégante plaquettaire, caractérisée en ce qu'elle comprend en combinaison de l'acide acétylsalicylique et de la taurine, ou leurs sels respectifs, dans un rapport en poids acide acétylsalicylique / taurine compris entre 1/5 et 1/50, en une quantité pharmacologiquement efficace, associés le cas échéant à un ou plusieurs excipients pharmaceutiquement acceptables.

2. Composition selon la revendication 1, caractérisée en ce que le rapport en poids de l'acide acétylsalicylique à la taurine est compris entre 1/10 et 1/30.

3. composition selon la revendication 2, caractérisée en ce qu'elle est sous forme de doses unitaires contenant 20 à 50 mg d'acide acétylsalicylique et 400 à 500 mg de taurine.

4. Utilisation d'une composition selon l'une quelconque des revendications précédentes pour la préparation d'un médicament pour le traitement des thromboses artérielles et artériolaires.

**Revendications pour les Etats contractants suivants : ES, GR**

1. Procédé de préparation d'une composition utile en thérapeutique, exerçant une activité anti-agrégante plaquettaire, caractérisé en ce qu'on mélange de l'acide acétylsalicylique et de la taurine, ou leurs sels respectifs, dans un rapport en poids acide acétylsalicylique / taurine compris entre 1/5 et 1/50, et on ajoute, le cas échéant, un ou plusieurs excipients pharmaceutiquement acceptables.

2. Procédé selon la revendication 1, caractérisé en ce que le rapport en poids de l'acide acétylsalicylique à la taurine est compris entre 1/10 et 1/30.

**Patentansprüche**

**Patentansprüche für folgende Vertragsstaaten : AT, BE, CH, DE, DK, FR, GB, IT, LI, LU, NL, SE**

1. In der Therapeutik anwendbare Zusammensetzung mit aggregationshemmender Wirkung auf Blutplättchen, dadurch gekennzeichnet, daß sie in Kombination Acetylsalicylsäure und Taurin oder deren entsprechende Salze enthält, in einem Gewichtsverhältnis Acetylsalicylsäure/Taurin von 1:5 bis 1:50 und in einer pharmakologisch wirksamen Menge sowie gegebenenfalls assoziiert mit einem oder mehreren pharmazeutisch annehmbaren Exzipienten.

2. Zusammensetzung nach Anspruch 1, dadurch gekennzeichnet, daß das Gewichtsverhältnis Acetylsalicylsäure/Taurin von 1:10 bis 1:30 beträgt.

3. Zusammensetzung nach Anspruch 2, dadurch gekennzeichnet, daß sie in Form von Einheitsdosierungen vorliegt, die 20 bis 50 mg Acetylsalicylsäure und 400 bis 500 mg Taurin enthalten.

4. Verwendung einer Zusammensetzung nach einem der vorhergehenden Ansprüche zur Herstellung eines Medikaments zur Behandlung arterieller und arteriolärer Thrombosen.

**Patentansprüche für folgende Vertragsstaaten : ES, GR**

1. Verfahren zur Herstellung einer in der Therapeutik anwendbaren Zusammensetzung mit aggregationshemmender Wirkung auf Blutplättchen, dadurch gekennzeichnet, daß Acetylsalicylsäure und Taurin, oder deren entsprechende Salze, in einem Gewichtsverhältnis Acetylsalicylsäure/Taurin von 1:5 bis 1:50 gemischt werden und gegebenenfalls ein oder mehrere pharmazeutisch annehmbare Exzipienten zugegeben wird bzw. werden.

2. Verfahren nach Anspruch 1, dadurch gekennzeichnet, daß das Gewichtsverhältnis Acetylsalicylsäure/Taurin von 1:10 bis 1:30 beträgt.

## Claims

### Claims for the following Contracting States : AT, BE, CH, DE, DK, FR, GB, IT, LI, LU, NL, SE

1. Composition useful in therapy, having a platelet anti-aggregating activity, characterized in that it comprises a combination of acetylsalicylic acid and taurine, or the respective salts thereof, with a weight ratio acetylsalicylic acid / taurine from 1:5 to 1:50, combined if necessary with one or several pharmaceutically acceptable carriers.

2. The composition of claim 1, characterized in that the weight ratio of acetylsalicylic acid to taurine is comprised between 1:10 and 1:30.

3. The composition of claim 2, characterized in that it comprises unitary doses containing from 20 to 50 mg of acetylsalicylic acid and from 400 to 500 mg of taurine.

4. The use of a composition according to any of the preceding claims for the preparation of a medicine for the treatment of arterial and arteriolic thrombosis.

### Claims for the following Contracting States : ES, GR

1. Process for the preparation of a composition useful in therapy, having a platelet anti-aggregating activity, characterized in that it comprises mixing acetylsalicylic acid and taurine, or the respective salts thereof, with a weight ratio acetylsalicylic acid / taurine from 1:5 to 1:50, and adding if necessary one or several pharmaceutically acceptable carriers.

2. The process of claim 1, characterized in that the weight ratio of acetylsalicylic acid to taurine is comprised between 1:10 and 1:30.